(19) Europäisches Patentamt / European Patent Office / Office européen des brevets

(11) **EP 4 516 215 A1**

(12) **EUROPEAN PATENT APPLICATION**

(43) Date of publication:
**05.03.2025 Bulletin 2025/10**

(21) Application number: **23194560.1**

(22) Date of filing: **31.08.2023**

(51) International Patent Classification (IPC):
*A61B 5/024* (2006.01)       *A61B 5/1455* (2006.01)
*A61B 5/00* (2006.01)

(52) Cooperative Patent Classification (CPC):
**A61B 5/14551; A61B 5/02416; A61B 5/02438;
A61B 5/7214; A61B 5/7246; A61B 5/7275**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **FRANCK, Christoph Florian
Eindhoven (NL)**
• **SZUTTOR, Kai
Eindhoven (NL)**
• **MOSS, Yvonne
5656AG Eindhoven (NL)**
• **HUCK, Salina Marie
Eindhoven (NL)**

(74) Representative: **Philips Intellectual Property &
Standards
High Tech Campus 52
5656 AG Eindhoven (NL)**

(54) **DEVICE, SYSTEM AND METHOD FOR DETERMINING PHYSIOLOGICAL INFORMATION OF A SUBJECT RELATED TO THE SUBJECT'S BLOOD**

(57)   The present invention relates to a device (32, 52), system (30, 40, 50) and method for determining physiological information of a subject related to the subject's blood. The device comprises circuitry (33) configured to obtain two or more photoplethysmography, PPG, signals of the subject in two or more different wavelength channels; determine the gradient or angle of a line in a two- or multidimensional coordinate system, in particular a cartesian coordinate system, fitting most PPG signal points in the coordinate system, wherein a PPG signal point is represented in the coordinate system by a tuple of PPG signal values of the two or more PPG signals acquired at substantially the same point in time; and determine the physiological information from the determined gradient or angle.

100

| OBTAIN PPG SIGNAL | 101 |
| DETERMINE GRADIENT / ANGLE OF LINE | 102 |
| DETERMINE PHYSIOLOGICAL INFORMATION | 103 |

**FIG.11**

EP 4 516 215 A1

## Description

FIELD OF THE INVENTION

The present invention relates to a device, system and method for determining physiological information of a subject related to the subject's blood, such as oxygen saturation and information on pathological modifications of hemoglobin.

BACKGROUND OF THE INVENTION

Pulse oximeters measure the oxygen saturation (also referred to as $SpO_2$) of a patient's arterial blood by utilizing the knowledge that only the blood flow in arteries is strongly pulsatile, and the oxygenated and deoxygenated hemoglobin have markedly different light absorption spectra. A pulse oximeter works by measuring the intensity of light of different wavelength ranges that has either passed through or is reflected by a volume of tissue with good blood supply. This results in a photoplethysmography (PPG) signal per wavelength range (which may cover a single wavelength or narrow wavelength band). By determining the amplitudes of the pulsatile part of each PPG signal, the pulse oximeter can estimate the ratio of oxygenated hemoglobin to total hemoglobin in the arterial blood.

Pulse oximetry is widely used in clinical practice, as it is non-invasive, safe, quick and convenient. A pulse oximeter often takes the form of a clip or sleeve sensor that is attached to one of the patient's fingers or another part of the body with good blood supply, such as an earlobe. It contains a light emitter (also called light source), generally two light emitting elements such as light-emitting diodes (LEDs), to generate and emit light of different, predefined wavelengths, and a light detector (also called sensor) opposite of the light emitter to measure the intensity of light that has passed through the tissue. The light emitting elements (e.g. LEDs) are commonly activated briefly in rapid sequence, and the pulse oximeter uses analog or digital signal processing (as processing unit) to process the single light intensity signal from the light detector into wavelength-specific light intensity signal channels (also called PPG signals).

More recently, pulse oximeters have also found application in consumer-grade devices like smart watches and fitness trackers.

The PPG signal for each wavelength contains undesired nuisance components, for example random noise from sensors and electronic components, artefacts caused by patient movements, and interference from ambient light sources or electromagnetic interference picked up by the pulse oximeter, the patient cable or the sensor. The presence of undesired components can make the determination of physiological information related to the subject's blood, e.g. the calculation of a ratio of ratios conventionally used for computing $SpO_2$, difficult and may reduce the accuracy, which in turn may causes the display and recording of incorrect physiological information, e.g. incorrect oxygen saturation values. The effects of undesired components can be exacerbated by health conditions like low perfusion or arrhythmia. Hence, there is a need for an improvement in the accuracy of the determined physiological information.

SUMMARY OF THE INVENTION

It is an object of the present invention to improve the accuracy of the determination of physiological information of a subject related to the subject's blood.

In a first aspect of the present invention device for determining physiological information of a subject related to the subject's blood is presented, the device comprising circuitry configured to:

- obtain two or more photoplethysmography, PPG, signals of the subject in two or more different wavelength channels;
- determine the gradient or angle of a line in a two- or multi-dimensional coordinate system, in particular a cartesian coordinate system, fitting most PPG signal points in the coordinate system, wherein a PPG signal point is represented in the coordinate system by a tuple of PPG signal values of the two or more PPG signals acquired at substantially the same point in time; and
- determine the physiological information from the determined gradient or angle.

In a further aspect of the present invention a device, system and method for determining physiological information of a subject related to the subject's blood is presented, the system comprising:

- a light detector configured to detect light in two or more different wavelength channels reflected from skin of a subject or transmitted through tissue of the subject in response and to generate two or more PPG signals from the detected light in the two wavelength channels; and
- a device as disclosed herein for determining the physiological information of the subject from the PPG signals.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to improve the accuracy of the determination of physiological information from PPG signals in two or more different wavelength channels, e.g. the ratio of magnitude variation, by using the assumption that the PPG intensity variations across multiple PPG wavelength channels are correlated, and that the irrelevant components are not correlated with the pulse component. The assumption of the pulse component consisting of a superposition of a small number of sinusoidal components is not made, and hence no errors due to this inaccurate model assumption are introduced. Further, with the present invention and its embodiments the determination of physiological information can be made robust against artefacts and outliers in the measured waveforms, which improves the availability and accuracy of the determined physiological information, e.g. blood oxygen saturation value, under adverse measurement conditions.

It shall be noted that with two light wavelength channels and two corresponding PPG signals blood oxygen saturation (also referred to as $SpO_2$) can be determined. With more than two light wavelength channels and more than two PPG signals, other information can be distinguished, such as pathological modifications of hemoglobin (e.g. carboxyhemoglobin (carbon monoxide bound to hemoglobin) and methemoglobin). This may be done by extending the basic pulse oximetry model to include additional light-absorbing pulsatile components in addition to oxyhemoglobin and deoxyhemoglobin. The additional components are additional unknown variables in the system of equations, while the additional wavelength channels yield additional equations. The additional wavelength channels thus correspond to additional angles in a higher-dimensional space. With three wavelength channels, two angles can be calculated, and each additional wavelength channel adds another angle. In practice, just like with basic $SpO_2$, a multi-wavelength pulse oximeter will likely use an empirical mapping function or mapping table that maps a tuple of angles to hemoglobin modification content relative to total hemoglobin (oxyhemoglobin/total hemoglobin, methemoglobin/total hemoglobin)

Thus, with this embodiment the coordinate system would be extended with additional dimensions, i.e., one dimension per wavelength channel. An ideal (noise-free) data set would still resemble a line in the multi-dimensional space. In a two-dimensional coordinate system, slope (as special case of gradient) of the line may be determined, while in the multi-dimensional coordinate system more generally the gradient (i.e. a vector) would be determined. Further, the ratios of the detected forms of hemoglobin may be determined from the values of the elements of the gradient.

The expression "tuple of PPG signal values" shall generally be understood as a set (or group or vector) of two or more PPG signal values. For instance, in case two wavelength channels and two PPG signals are used, a tuple of PPG signal values means a pair of (i.e. two) PPG signal values. In case of e.g. four wavelength channels and four PPG signals, a tuple of PPG signal values means a set of four PPG signal values. The property of a tuple is that it is an ordered list, i.e. the position of an entry carries a meaning. In the present case, the position denotes which wavelength channel the PPG signal value belongs to.

The gradient or angle of a line is preferably determined in a two- or multi-dimensional cartesian coordinate system fitting most PPG signal points in the coordinate system. A cartesian coordinate system can have more than two axes. Since the measurements are in the form of tuples, they are naturally in cartesian coordinates. Representing the same data using different coordinates (e.g. polar) requires calculations for the transformation. For instance, a Hough transform is basically about transforming points from a cartesian representation to one in which the shapes of interest (e.g. lines or circles) are points. Hence, the step of determining a gradient or angle may generally also be performed in another coordinate system.

According to an embodiment the circuitry is configured to use a statistical method to determine the gradient or angle of the line. It has been found that most components of the PPG signals have a statistical value distribution that is not a normal/Gaussian distribution. This allows the reconstruction of individual components via statistical techniques, such as (regular, L2-norm) principal component analysis (PCA), Lp-norm principal component analysis, and independent component analysis (ICA).

According to another embodiment the circuitry is configured to use one of the following methods to determine the one or more parameters of the line and/or the gradient or angle of the line:

- line fitting;
- principal component analysis, PCA;
- Lp-norm PCA;
- general projection pursuit using utility and/or penalty functions;
- independent component analysis, ICA;
- maximum likelihood estimation;
- Hough transform; and
- random sample consensus, RANSAC.

It was recognized that determining e.g. a ratio of ratios, as done for determining $SpO_2$, corresponds to searching for a line in the two-dimensional cartesian coordinate system of PPG signal values in a first wavelength channel (e.g. red light) versus PPG signal values in a second wavelength channel (e.g. infrared (IR) light) (also called red/IR plane). This allows the application of line detection methods (e.g. line fitting, random sample consensus or the Hough transform, etc.). The proposed methods determine or estimate the parameters of a line, which could be a (gradient, intercept) tuple, or just the gradient (or slope) if the intercept is assumed to be zero. Alternatively, the methods could estimate an angle and an intercept. This may be mathematically preferable, as angles are within a bounded range and exist even for cases where the gradient / slope is not defined (i.e. if there is an infinite slope).

Thus, from the output of a line fitting/line estimation/line approximation step, a ratio of ratios may be determined. The skilled person will generally be familiar with the general operation of the methods mentioned in the list above and generally understands how they are used to determine parameters of a line from input that consists of a set of points.

The circuitry may further be configured to take the logarithm of the PPG values and to represent a PPG signal point in the coordinate system by a tuple of the logarithm of the PPG signal values of the two or more PPG signals acquired at substantially the same point in time. Taking the logarithm may be the first step to calculate the "ratio of difference of logarithms" or its approximation, the "ratio of ratios". Without taking the logarithm (or dividing by the DC component) the signal of interest will be a fraction of the large DC component. Taking the logarithm (or dividing by the DC component) at this point is useful but not mandatory.

The circuitry may advantageously be configured to use the determined gradient or angle as ratio of ratios or determine the ratio of ratios from the determined gradient or angle and determine oxygen saturation of the subject's blood based on the ratio of ratios, in particular using a calibration table between ratio of ratios values and values of oxygen saturation. This basically corresponds to the conventional way of determining $SpO_2$, which will be more accurate through the use of the present invention.

The PPG signals may be pre-processed by high-pass filtering and/or low-pass filtering before representing the PPG signal points in the coordinate system and determining the gradient or angle of the line. This may remove a mean value of the PPG signals and thus further improve the accuracy of the determined physiological information.

In an embodiment the circuitry is configured to non-uniformly sample the PPG signals to obtain the PPG signal values represented in the coordinate system. For instance, it may be determined which sections of the PPG wave contain more information (such as the sections containing the extreme points) and which sections contain less information (such as the sections between extreme points). The sampling rate may thus be reduced (or one of the light sources and/or sensors may be deactivated) during less interesting sections in order to save power. Driving the light sources amounts to a significant part (up to 50%) of the total power consumption of a pulse oximeter so that reducing the time the light sources are active can yield significant power savings.

In another embodiment the circuitry is configured to partition the PPG signals into PPG signal segments and to determine the gradient or angle of a line in the coordinate system and to determine the physiological information per tuple of PPG signal segments for the two or more wavelength channels. This may further improve the accuracy and/or reduce computational load.

In still another embodiment the circuitry is configured to

- determine the gradient or angle of the line by use of two or more different methods;
- determine the physiological information from each of the determined gradients or angles; and
- determine quality information indicating the quality of the determined physiological information based on the discrepancy of the physiological information values determined from the determined gradients or angles.

Unreliable physiological information determined by one of several methods may then be ignored or less weighted in the determination of the physiological information e.g. as a weighted average of the different physiological information values. Further, the user may be informed of the quality of the physiological information and may thus decide to ignore or use it depending on the determined quality information.

According to another embodiment the circuitry is configured to determine quality information indicating the quality of the determined physiological information based on

- residual error values, or
- a ratio of eigenvalues of a covariance matrix to a largest eigenvalue, or
- a ratio of the variance of non-pulse signal components to a variance of a PPG signal.

As mentioned above already, the physiological information may be oxygen saturation, but information on pathological modifications of hemoglobin of the subject's blood may be determined from the determined slope gradient or angle as well.

The system of the present invention comprises a light detector for detecting light in two or more different wavelength channels reflected from skin of a subject or transmitted through tissue of the subject in response and to generate two or more PPG signals from the detected light in the two wavelength channels. The light detector may e.g. be a camera that is configured to remotely detect light from skin of the subject, e.g. a camera mounted to the wall or ceiling for monitoring the subject.

Alternatively, the light detector may be a light sensor that is configured to be arranged in contact with or close to skin of the subject. In this case the system further comprises a light emitter that is configured to emit light in the two or more wavelength channels, e.g. an LED unit comprising two LEDs (e.g. one emitting red light and the other one emitting IR light). The light detector and the light emitter may then be configured as a wearable, such as wristband or chest belt device or another device that can be worn by the subject. Further, the whole system may be implemented as a body-worn device, similar to a pulse oximeter, e.g. in the form of a clip that can be clipped to a body part (e.g. finger, ear lobe, nasal alar, toe) or in the form of a wristband or chest belt.

BRIEF DESCRIPTION OF THE DRAWINGS

These and other aspects of the invention will be apparent from and elucidated with reference to the embodiment(s) described hereinafter. In the following drawings

   Fig. 1 shows a diagram illustrating a red intensity waveform and an IR intensity waveform without artefacts.
   Fig. 2 shows a joint representation of the red and IR waveforms shown in Fig. 1.
   Fig. 3 shows a projection of the data of the red and IR waveforms shown in Fig. 1 on the red/IR plane as data points.
   Fig. 4 shows a diagram illustrating a red intensity waveform having an artefact and an IR intensity waveform.
   Fig. 5 shows a joint representation of the red and IR waveforms shown in Fig. 4.
   Fig. 6 shows a projection of the data of the red and IR waveforms shown in Fig. 4 on the red/IR plane as data points.
   Fig. 7 shows a schematic diagram of a first embodiment of a system according to the present invention.
   Fig. 8 shows a schematic diagram of an exemplary implementation of a system according to the present invention.
   Fig. 9 shows a schematic diagram of an embodiment of a device according to the present invention.
   Fig. 10 shows a schematic diagram of another exemplary implementation of a system according to the present invention.
   Fig. 11 shows a schematic diagram of an embodiment of a method according to the present invention.

DETAILED DESCRIPTION OF EMBODIMENTS

Oxygen saturation is the ratio of oxygenated hemoglobin to total hemoglobin in the blood. It is a parameter that allows detection of conditions like insufficient oxygen content in the inspired air, insufficient respiratory rate or respiratory tidal volume, or insufficient oxygen transfer from lung volume to blood.

Oxygenated hemoglobin and deoxygenated hemoglobin have different light absorption spectra. Pulse oximetry uses this property and the assumption that only the blood flow in arteries is pulsatile to isolate the arterial blood oxygen saturation from the saturation in other blood vessel types without pulsatile flow. A pulse oximeter transmits light with multiple wavelengths through a part of the patient's body with sufficient transparency and blood flow, such as a finger or ear lobe, and records the light intensity of each wavelength on the receiver's side over time in the form of wavelength intensity channel signals. Each channel will contain a signal that varies over time due to the pulsatility of the blood flow, as each channel contains a photoplethysmogram (PPG). The magnitude of the variation in each channel will depend on the light absorption of the arterial blood. The pulse oximeter can then derive the ratio of the magnitude variation of two channels and convert the ratio to an oxygen saturation percentage ($SpO_2$), usually by using a look-up table derived from empirical data.

In addition to the blood oxygen saturation value, pulse oximeters commonly also calculate the patient's pulse rate from the PPG signals, as the pulse rate is a useful vital parameter, and it is readily derived from an available PPG signal.

The PPG signal for each wavelength contains undesired nuisance components, for example random noise from sensors and electronic components, artefacts caused by patient movements, and interference from ambient light sources or electromagnetic interference picked up by the pulse oximeter, the patient cable or the sensor. The presence of undesired components can make the calculation of the ratio difficult and reduce the accuracy of the ratio value, which in turn causes the pulse oximeter to display and record incorrect oxygen saturation values. The effects of undesired components can be exacerbated by health conditions like low perfusion or arrhythmia.

The ability of a pulse oximeter to derive an accurate value for the blood oxygen saturation even under adverse conditions with significant undesired components in the PPG signals is an important quality criterion for the user and subject to intensive research.

The combination of ease of use and the recording of a useful physiological parameter makes pulse oximetry an important and highly visible component of a patient monitoring system. With the availability of consumer-grade pulse oximeters and the integration of pulse oximeters into smart watches, applications outside the clinical field are increasing.

Both the blood oxygen saturation value and the pulse rate calculated by a pulse oximeter are sensitive to undesired, irrelevant components on the PPG waves. Artefacts are primarily caused by patient motion, but they can also be due to sudden changes in the ambient light level. Continuous interference can happen due to artificial ambient light sources, or due to electromagnetic interference coupling into the sensor, the cable or the pulse oximeter itself. Noise is a property of the light sources, the light sensor(s) and of electronic components like resistors, amplifiers and analog-to-digital converters (ADC) used to transmit and process the signal for driving the light sources and the light intensity signal of the sensor(s). When the PPG signals contain a significant amount of undesired components in relation to the pulse component, the blood oxygen saturation and the heart rate value may become inaccurate or unavailable. Inaccurate values may mimic or mask pathological conditions, resulting in unnecessary or delayed treatment, respectively. Unavailability of the numeric values reduces the confidence of the user in the device and may also result in delayed treatment of a pathological condition.

Current pulse oximeters employ techniques like analog and digital filtering of the light intensity signal to reduce the undesired components and maintain availability and accuracy of the saturation and pulse rate values. There are also a number of more complex algorithms, such as Philips' Fourier Artefact Suppression Technology (FAST), which employ techniques beyond filtering, such as frequency-domain transformations or autocorrelation, in order to reduce the effects of undesired components.

Such algorithms often assume a model in which the pulse signal of interest can be represented as a superposition of a small number of sinusoids of different frequencies (in case of frequency-domain techniques), or as a sequence of repeated, similar signal segments (in case of autocorrelation). This assumption is only valid for periods during which the beat-to-beat heart rate and cardiac stroke volume is nearly constant. However, even in a healthy patient the heart rate varies slightly from beat to beat. In patients with cardiac conditions like arrhythmias, the heart rate and the stroke volume may change drastically from beat to beat, resulting in a PPG wave with irregular appearance. This will affect frequency bin amplitudes calculated by a discrete Fourier transform (DFT) and also the correlation bin amplitudes of an autocorrelation. Generally, instead of sharp, defined peaks, peaks will be flatter and wider, taking on a less distinct, "fuzzy" appearance, which reduces the signal-to-noise ratio.

With the present invention, the accuracy of the ratio of magnitude variation is provided by using the assumption that the PPG intensity variations across multiple PPG wavelength channels are correlated, and that the irrelevant components are not correlated with the pulse component. The assumption of the pulse component consisting of a superposition of a small number of sinusoidal components is not made, and hence no errors due to this inaccurate model assumption are introduced. Further, the present invention improves robustness against artefacts and outliers in the measured waveforms, which improves the availability and accuracy of the blood oxygen saturation value or other physiological information under adverse measurement conditions.

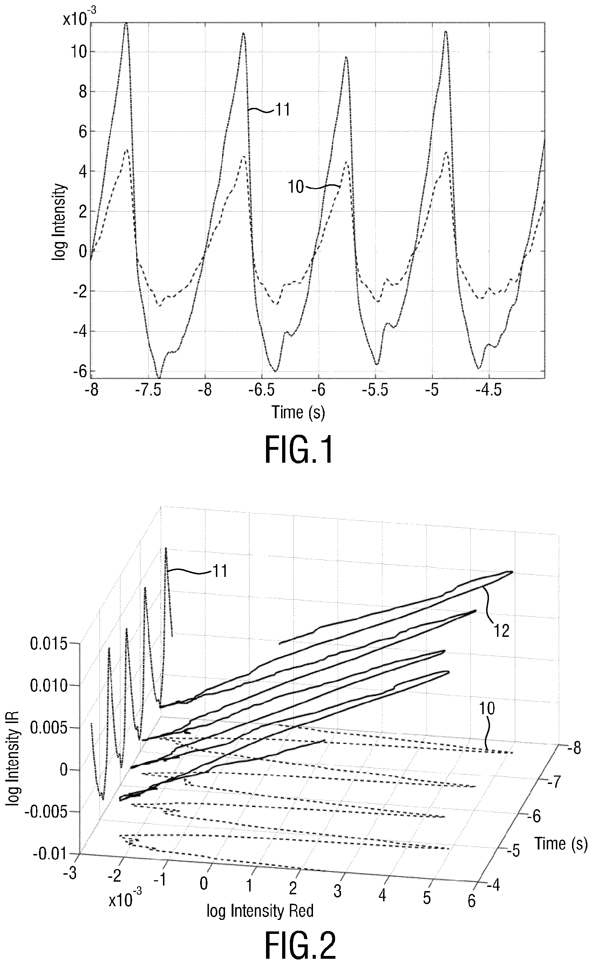

In general, pulse oximeters acquire light intensities of at least two wavelength channels, usually a red wavelength channel and an infrared (IR) wavelength channel. Fig. 1 shows a diagram illustrating the logarithm of the intensity in the red wavelength channel, also called red intensity waveform 10, and in the IR wavelength channel, also called IR intensity waveform 11, over time (in seconds). The light intensity wave samples are processed, for example by high-pass and/or low-pass filtering, and then used to calculate either a "ratio of ratios" or a "ratio of differences of logarithms" value. In this common approach, the joint properties of the intensity samples, such as their correlation, are not considered at all, or only considered during the later steps of the algorithm. If the joint properties are not considered, the algorithm may not recognize abnormal conditions, like the intensity waveforms being uncorrelated due to interference or defective equipment.

For typical wavelengths (e.g. red 660 nm, IR 900 nm), the ratio is typically between 0.4 (corresponding to 100% SpO$_2$) and 2.5 (corresponding to 20% SpO$_2$). This corresponds to angles of slope of about 20° to about 70°.

Fig. 2 shows a joint representation 12 of the red intensity waveform and the IR intensity waveform. In this representation 12, it becomes clear that the "ratio of differences of logarithms" (or its approximate, the "ratio of ratios") is equivalent to the direction of the main axis of the set of data points in the red/IR plane.

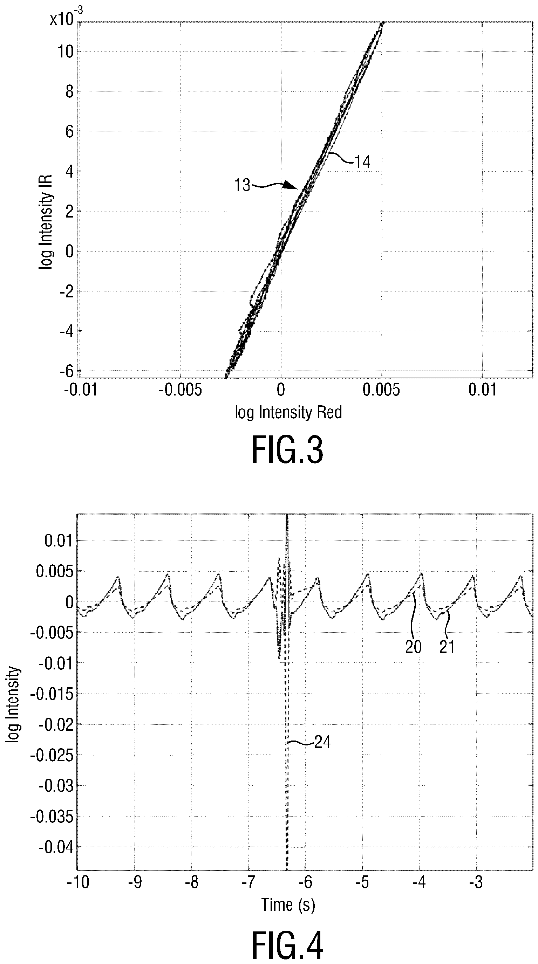

Fig. 3 shows a projection 13 of the data of the red and IR waveforms 10, 11 on the red/IR plane as data points of (red, IR). The direction of the main axis 14 (or alternatively the slope/gradient of an imagined line fitted to the set of data points) can be seen clearly, as no artefacts and only small amounts of noise are present. To determine the direction of the main axis, various methods may be applied, such as line fitting, principal/independent component analysis, random sample consensus, and maximum likelihood estimation.

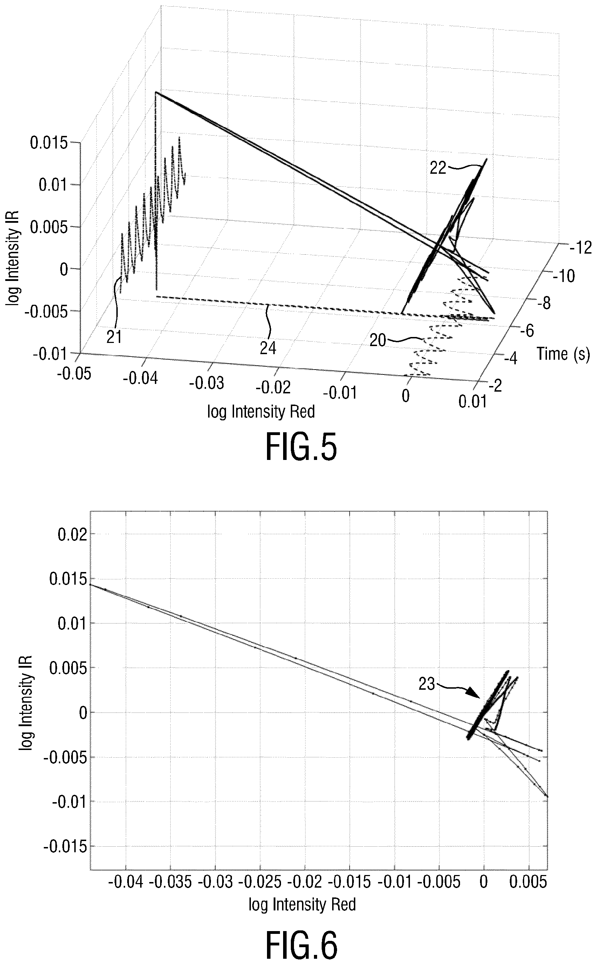

Figs. 1-3 show clean signals for demonstration purposes. Deriving the ratio or slope from the intensity waveforms may not be very challenging. Figs. 4-6 show signals with an obvious artefact that may occur in practice. In particular, Fig. 4 shows a diagram, similar to Fig. 1, illustrating a red intensity waveform 20 and an IR intensity waveform 21. The artefact 24 is clearly visible. Fig. 5 shows, similar to Fig. 2, a joint representation 22 of the red intensity waveform 20 and the IR intensity waveform 21. Fig. 6 shows, similar to Fig. 3, a projection 23 of the data of the red and IR waveforms 20, 21 on the red/IR plane as data points of (red, IR). With the present invention, it will be possible to derive the desired physiological information, such as SpO$_2$ values, with sufficient accuracy even in the presence of artefacts.

The present invention is based on the insight that PPG signals are not described optimally by models that assume that the signals consist of a superposition of a small number of sinusoids. A further insight used in this invention is that the different components of the PPG wave, i.e. the pulse rate component, motion artefact components and ambient light components, are not or only weakly correlated with each other, but are strongly correlated across the individual PPG wavelength channels. A third insight is that most components of the PPG signals have a statistical value distribution that is not a normal/Gaussian distribution.

This allows the reconstruction of individual components via statistical techniques like (regular, L2-norm) principal component analysis (PCA), Lp-norm principal component analysis, and independent component analysis (ICA). Further, determining the ratio corresponds to searching for a line in the red/IR plane, which allows the application of line detection methods (e.g. line fitting, random sample consensus or the Hough transform).

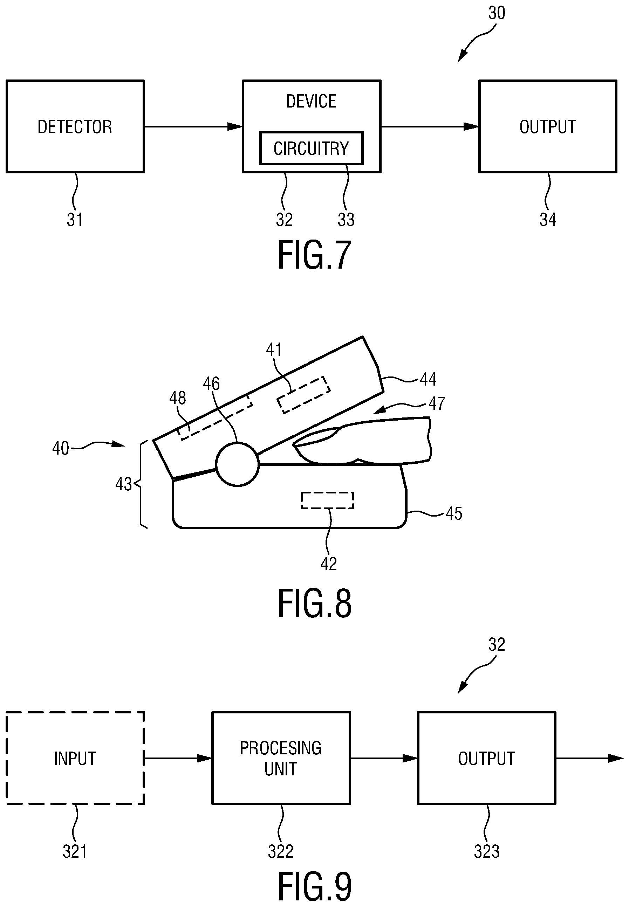

Fig. 7 shows a schematic diagram of an embodiment of a system 30 for determining physiological information of a subject related to the subject's blood, e.g. for determining a vital sign such as the blood oxygen saturation (SpO$_2$) of a subject. The system 30 comprises a light detector 31 configured to detect light in two or more different wavelength channels reflected from skin of a subject or transmitted through tissue of the subject and to generate two or more PPG signals from the detected light in the two wavelength channels. The system 1 further comprises a device 32 for determining the physiological information of the subject from the PPG signals as will be explained in more detail below.

The device 32 may be implemented by respective units or circuitry 33, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor may represent the device 32, which may execute a computer program that may be stored in a memory that is accessed by the processor.

Optionally, the system 30 may further comprise an output interface 34 configured to issue the determined. The optional output interface 34 may generally be any means that outputs the determined physiological information in visual or audible form, e.g. in text form, as image or diagram, as sound or spoken words, etc. For instance, the output interface 34 may be a display, a loudspeaker, a touchscreen, a computer monitor, the screen of a smartphone or tablet, etc. In an embodiment, the output interface 34 may be a separate device or may be combined with the detector 31 and/or the device 32, e.g. may be integrated into the housing of the detector 31 and/or the device 32, for instance in the form of a display within or on top of a surface of the housing.

Fig. 8 shows a side view of a pulse oximeter 40 in an open state, representing an exemplary implementation of the system 30. In this exemplary embodiment the pulse oximeter 40 is configured substantially in the same manner as a conventional pulse oximeter in the form of a finger clip that can be clipped at the subject's finger. The pulse oximeter 40 has a housing 43 formed by two housing portions 44, 45 that are hinged together by a swivel joint 46. Between the two housing portions 44, 45 a measurement location 47 (also called measurement area) is formed.

Light is emitted by the light emitter 41 towards the measurement location, which is the part (in this example the fingertip) of the subject's anatomical object (in this example the finger) that is arranged in the measurement area 47. The light detector 42 detects light that has, after emission from the light emitter 41, transmitted through the subject's tissue (i.e., the fingertip) at the measurement location 47. Further, a display 48 is arranged within the housing portion 14, e.g. to display the computed physiological information.

In other embodiments the pulse oximeter 40 may be configured for arrangement at other anatomical objects, such as the ear lobe or the nasal alar. It may be configured as clip for clipping it to the anatomical object, as shown in Fig. 8, or as wearable, e.g. in the form of or integrated into a smartwatch, belt or other means that can be mounted (e.g. taped) to the subject's body. Particularly in sensors formed as wearables, the light detector 42 may be configured to detect light that has, after emission from the light emitter 41, been reflected from the subject's tissue, in particular the subject's skin. Thus, both the light emitter 41 and the light detector 42 may be arranged within the same housing portion and may be a placed next to each other.

The light emitter 41 is configured to emit light in two or more different wavelength ranges, e.g. near-infrared light and red light. It may comprise two separate LEDs that emit the respective light in the two (or more) wavelength channels, either simultaneously or alternately.

The light detector 42 is configured to detect light in two or more different wavelength channels reflected from skin of the subject or transmitted through tissue of the subject in response to irradiation by the light emitter 41. Each wavelength channel covers one of the two (or more) different wavelength ranges. The light detector 42 comprises a single photosensor unit that is sensitive to light in a broad wavelength range or only in the wavelength ranges of interest in which light is emitted by the light emitter (e.g. red light and near-infrared light). In other embodiments separate photodiodes may be used which are each sensitive for light in different wavelength channels (e.g. one photodiode for red light and another photodiode for near-infrared light).

From the detected light in the respective wavelength channels two (or more) PPG signals are generated. A PPG signal represents the intensity of the detected light over time in a wavelength channel of interest. For instance, two PPG signals in the red and near-infrared wavelength channels may be derived from the detected light. This may be done by the light detector 42, which may comprise processing circuitry to process the detected light accordingly. In other embodiments this processing may be done in the device 32.

The device 32 is preferably combined with the pulse oximeter 40, e.g. is integrated into the housing 43 of the pulse oximeter 40. In other embodiments the device 32 may be a separate device. Generally, the PPG signals acquired by the pulse oximeter 40 may be provided to the device 32 through a wired or wireless connection.

Fig. 9 shows a schematic diagram of another embodiment of a device 32 for operating the pulse oximeter according to the present invention. In this embodiment, the device 32 comprises a processing unit 322 that processes the PPG signals as explained in more detail below.

Optionally, an input 321 may be provided to obtain the PPG signals from the light detector 31. The input 321 may be directly coupled or connected to the light detector 31 or may obtain (i.e. retrieve or receive) the PPG signals from a storage, buffer, network, or bus, etc. The input 321 may thus e.g. be (wired or wireless) communication interface or data interface, such as a Bluetooth interface, Wi-Fi interface, LAN interface, HDMI interface, direct cable connect, or any other suitable interface allowing signal transfer to the device 20.

Further, the device 32 may optionally comprise an output 323 configured to output the determined physiological information. The output 323 may generally be any interface that provides the determined physiological information, e.g. transmits it to another device or provides it for retrieval by another device (e.g. a smartphone, computer, tablet, etc.), or simply outputs it to a display, e.g. the output interface 34 or the display 48. It may thus generally be any (wired or wireless) communication or data interface.

The processing unit 322 may be any kind of means configured to process the PPG signals and determine the physiological information. It may be implemented in software and/or hardware, e.g. as a programmed processor or computer or app on a user device such as a smartphone, smartwatch, tablet, laptop, PC, workstation, etc.

The processing unit 322 (and, optionally, the input unit 321 and the output unit 323) may together form the circuitry 33 of the device 32.

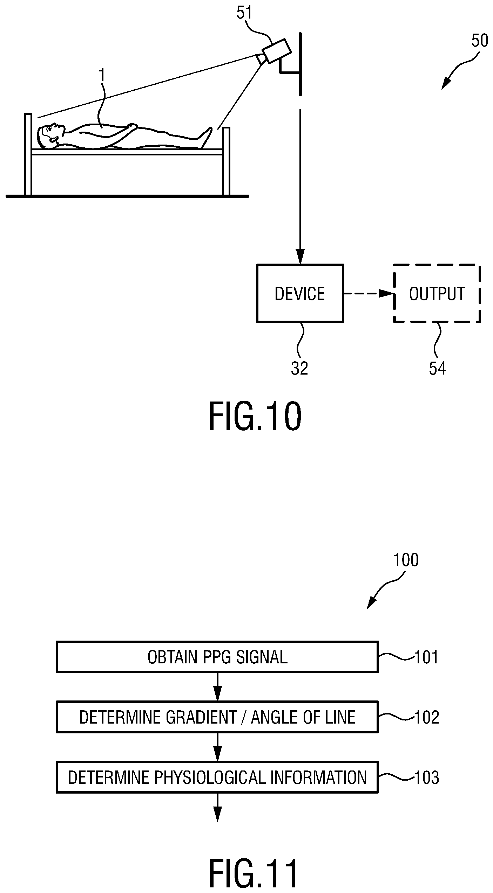

Fig. 10 shows a schematic diagram of another embodiment of a system 50 for determining a physiological information of a subject 1, e.g. patient in a hospital bed or an elderly person at home or in a rest home. The system 50 comprises an imaging unit 51 for monitoring the subject and acquiring image data of the subject 1. The system 50 further comprises a device 52 and, optionally, an output interface 54 that are generally configured and operating as discussed above for the device 32 and the output interface 34. In a practical implementation, the device 52 and the output interface 54 may be implemented as or integrated into a patient monitor or other monitoring equipment.

The imaging unit 51 represents the light detector and may be a camera to record images (e.g. subsequent still images or video data) continuously or periodically. For instance, a surveillance camera, webcam or pan-tilt-zoom (PTZ) camera may be used.

The device 52 may generally be configured in the same way as the device 32 as shown in Fig. 9, i.e., it may comprise circuitry or other processing means or units to carry out the desired operations.

The system 50 of this embodiment makes use of the known remote PPG technology, by which PPG signal are extracted from remotely acquired images of the subject 1.

Fig. 11 shows a schematic diagram of an embodiment of a method 100 for determining physiological information of the subject according to the present invention. The steps of the method may be carried out by the device 32 or 52, wherein the main steps of the method are carried out by the processing unit 322. The method may e.g. be implemented as computer program running on a computer or processor.

In a first step 101, two or more PPG signals of the subject in two or more different wavelength channels (i.e., one PPG signal per wavelength channel) are obtained. For instance, a first PPG signal in the red wavelength range and a second PPG signal in the IR wavelength range are obtained. The PPG signals may e.g. be obtained directly from a light detector 42 of a pulse oximeter 40 or from an imaging unit 51 that remotely monitors the subject 1 and derives the PPG signal from the acquired image data. In an embodiment, the device 52 (i.e., not the light detector 31 or the imaging unit 51) derives the PPG signals from the sensor signals acquired by the light detector 31 or the image data acquired by the imaging unit.

In a second step 102, the gradient or angle of a line in a two- or multi-dimensional cartesian coordinate system fitting most PPG signal points in the coordinate system is determined. A PPG signal point is represented in the coordinate system by a tuple (i.e. a group or set) of PPG signal values of the two or more PPG signals acquired at substantially the same point in time. Hence, if e.g. four PPG signals of different wavelength channels are used, the tuple comprises four PPG signal values (each from a different PPG signal, but at substantially the same time instant). These four PPG signal values each represent a different dimension in a four-dimensional coordinate system.

In a third step 103, the physiological information is determined from the determined gradient or angle. For instance, the gradient (or slope) of the determined line represents the ratio of ratios commonly used for determining the subject's current $SpO_2$ value.

The determined physiological information (and, optionally, the PPG signals) may then be output, e.g. displayed on a display or transmitted to another entity, e.g. a patient monitor or central monitoring station or a mobile device (tablet, smartphone, etc.) of a caregiver.

Thus, in a practical embodiment as e.g. shown in Fig. 8, a pulse oximeter acquires light intensity signals of multiple (two or more) wavelength channels. The logarithm of the light intensity values is taken to convert the multiplicative light absorption model into a log-additive model as used in the well-known Beer-Lambert absorption law used for pulse oximetry. Alternatively, the logarithm can be linearized to arrive at the ratio of ratios expression. Software in the pulse oximeter processes the PPG signals to remove the mean value, for example by using a high-pass filter. Optionally, other preprocessing, such as low-pass filtering, is applied to the PPG signals. Unlike time- and frequency-domain techniques for calculating the ratio, low-pass filtering may only have limited benefits or be detrimental to the performance of statistical estimation techniques, as it alters the statistical value distribution of the undesired signal components.

After preprocessing, the wavelength log-intensity or linearized-log-intensity signals are stored in a matrix X, with each column of X corresponding to one wavelength channel and each row of X corresponding to samples taken at the same point in time:

$$\mathbf{X} = \begin{bmatrix} x_{red}(n) & x_{ired}(n) \\ x_{red}(n+1) & x_{ired}(n+1) \\ x_{red}(n+2) & x_{ired}(n+1) \\ ... & ... \end{bmatrix}$$

Based on these preprocessed signals, there exist multiple options to carry out the subsequent steps of determining a line and its gradient/slope or angle and of determining the desired physiological information from this gradient/slope or angle.

According to a first option, a ratio calculation by line fitting is used. A linear function is fitted to the set of (e.g. red and IR) data points by minimizing the residual errors between the fitted line and the set of data points. The fitting may be limited to a line with zero offset if the intensity signals were high-pass filtered before. The fitting should be done using orthogonal regression (or, if information about the noise variance is known or can be estimated, the more general Deming regression), as all intensity signal channels are subject to noise. Different penalty functions may be applied to the error terms during the fitting process, such as least-squares (mathematically simple, closed form solution, but not robust against outliers), other Lp-norms of the error (especially the L1 norm, which is robust against outliers), or penalty functions that are not norms like the Huber loss function or the log cosh function (which are approximations of the L1 norm while having a continuous derivative, but they are not scale-invariant). Penalty functions of the error terms e that grow slower than linear, e.g. using *sqrt(abs(e + ε))* or *-exp(-(e²))* are even more robust but result in mathematically more difficult (non-convex) optimization problems. The ratio is then given directly by the slope of the fitted line. The residual error at the end of the fitting process may additionally be used to derive a signal quality indicator.

According to a second option, a ratio calculation by principal component analysis (PCA) is used. A (regular, L2-norm) PCA is performed on the matrix X. A regular PCA finds signal components of the input signals that are correlated. The input signals are decomposed into principal components that are not correlated with each other (i.e., the principal components are decorrelated). Further, the principal component corresponding to the pulse signal are identified. Usually, it will be the component with the greatest variance. The ratio can be determined from the weighting coefficient of the vector by dividing the weight of the IR channel by the weight of the red channel. The principal component analysis can be performed using different algorithms. Finding the eigenvectors of the covariance matrix is the usual method, but the principal components can also be found by using projection pursuit to iteratively find directions with maximum variance. If only two wavelength channels are used, finding the first maximum variance direction also gives the second principal component, which is orthogonal to the first.

According to a third option, a ratio calculation by Lp-norm PCA is used. An Lp-norm ($1 \leq p < 2$) is performed on the matrix X. Lp-norm PCA usually does not achieve full decorrelation but is increasingly robust against outliers as lower values of p are chosen. L1-PCA is preferred, as is the most robust option. The Lp-principal component corresponding to the pulse signal is determined. As with regular PCA, it is usually the component with the greatest variance, and the ratio can be determined by dividing the weight of the IR channel by the weight of the red channel. Lp-norm PCA may have closed-form solutions in some cases, but it can also be performed using iterative projection pursuit to find directions with maximum Lp-norm that are orthogonal to any directions found previously. In case of only two wavelength channels, only one iteration of projection pursuit is necessary.

According to a fourth option, a ratio calculation by general projection pursuit using utility/penalty functions is used. The linear combination (with the L2 norm of the linear combination coefficients being constrained to be equal to a constant) of the matrix X that maximizes a utility function that is not a norm is found. *log cosh* can be used as a utility function, or the Huber loss function can be used as a utility function. The pulse signal corresponds to the component given by the linear combination if the signal-to-noise ratio is sufficiently good. The ratio can again be determined by dividing the weighting coefficient of the IR channel by the weighting coefficient of the red channel. Generally, this method is performed by projection pursuit using the respective utility function. If only two wavelength channels are used, finding the first major direction is sufficient, as the second major direction is orthogonal to the first. If more than two wavelength channels are used, multiple passes of projection pursuit will find the major directions, where each direction is orthogonal to all previously found directions.

According to a fifth option, a ratio calculation by independent component analysis (ICA) is used. An independent component analysis is performed on the matrix X, preferably using a contrast function that does not rise faster than linearly for robustness. For ICA algorithms that return the transformation matrix from input data to independent components, the independent component corresponding to the pulse signal are identified. The ratio can be determined from the weighting coefficients (usually a column of the transformation weight matrix) by division. If the ICA algorithm works on pre-whitened data and the transformation matrix of the pre-whitening step is not known, the ratio can be determined by synthesizing the feature vector corresponding to the pulse rate signal and dividing the dot product of one wavelength channel with the feature vector by the dot product of the other wavelength channel with the feature vector.

According to a sixth option, a ratio calculation by maximum likelihood estimation or a reduced Hough transform, reduced to slope-only/angle-only (as the line of red/IR goes through the origin) is used. The reduced Hough transform (lines through the origin only, i.e., the only parameter is the slope or angle of a line passing through the origin) is calculated using the matrix X and a suitable probability density for voting. Preferred choice is the Laplace distribution, but the standard Hough transformation uses yes/no voting which corresponds to a uniform probability density. If yes/no voting is used, kernel density estimation with a suitable kernel can be applied to the result to smoothen it and remove local maxima and plateaus). The maximum of the reduced Hough transform is found. This can be done using an optimization algorithm, or by exhaustive search over a sufficiently dense grid of points. The tangent or cotangent of the angle at which the maximum occurs directly gives the ratio. Alternatively, the pulse oximeter may directly convert the angle to an $SpO_2$ value. This requires using a function mapping angle to $SpO_2$ instead of the usual mapping of a ratio to $SpO_2$.

For each slope or angle $\theta$, the Hough transform either determines, for a sufficiently dense grid of $\theta$, the number $L(\theta, X)$ of rows (1... N) of X that vote (0 or 1) for a line through the origin passing through the points and optionally applies kernel density estimation to smoothen the function mapping a slope/angle to vote numbers. Whether a row of X votes for a particular slope/angle is given by the voting function $v(\theta, red, IR)$, with the red and IR being taken from one row of X.

$$L(\theta, \mathbf{X}) = \sum_{i=1}^{N} v\big(\theta, \quad \mathbf{X}_{1,N}, \mathbf{X}_{2,N}\big)$$

If the grid of $\theta$ is uniformly spaced, applying kernel density estimation is equivalent to convolution of L with the kernel k:

$$L_{KDE}(\theta, \mathbf{X}) = \mathbf{k} * L(\theta, \mathbf{X})$$

Alternatively, a fuzzy voting function $fv(\theta, red, IR)$ may be used which maps the input to value between zero and a constant (for convenience, the constant may be 1.0; scaling by a positive factor does not change the results of the voting):

$$L(\theta, \mathbf{X}) = \sum_{i=1}^{N} fv\big(\theta, \quad \mathbf{X}_{1,N}, \mathbf{X}_{2,N}\big)$$

The slope/angle $\theta_{ML}$ corresponding to the most likely line is then found as

$$\theta_{ML} = \arg \max_{\theta} L(\theta, \mathbf{X})$$

or respectively, if using kernel density estimation,

$$\theta_{ML} = \arg \max_{\theta} L_{KDE}(\theta, \mathbf{X})$$

If the logarithm of the intensity signals was used and the signals are not high-pass filtered, the line will not pass through the origin. In this case, the full Hough transform (which uses the intercept as a coordinate in addition to slope/angle) may be used, but this is computationally intensive.

According to a seventh option, a ratio calculation by random sample consensus / RANSAC is used. The best fitting line to the data points is found using the random sample consensus method. This comprises iteratively performing the steps of selecting a (small) random subset of the data points, optimally fitting the model function (e.g. line through the origin for the ratio slope) to the random subset, and then assessing the agreement of all of the data points with the fitted model by applying a voting function. If the agreement with the model is better than the best agreement found by previous iterations, the current model parameter set is considered the best fit, otherwise the best previous model parameter set is retained. The ratio can be determined directly from the slope of the best fit model.

Optionally, the number of data points for RANSAC may be reduced by dividing the light intensity signals into segments of several samples and calculating a slope for each segment. This assumes that erroneous values are not randomly distributed across the signals but appear in groups. Reducing the number of data points increases the speed of RANSAC and may even allow an exhaustive search of all possible random samples of a given size.

The selection of the random sample may also take the assumed grouping of erroneous samples into account by ensuring that the rows of the matrix X selected for the random sample are at least a certain number of rows apart. This reduces the size of the set of possible random samples. Additionally, also under the assumption that samples affected by large artefacts are grouped, the random sample may be drawn from a sequence of samples that is shorter than the total data set. The length and position of the sequence may be chosen separately for each iteration of RANSAC.

Standard RANSAC uses a voting function that classified the data points as inliers or outliers, which provides robustness by limiting the contribution of individual data points. The method may use different loss functions for fitting a line to the random sample, and for assessing the goodness of fit of the line to all data points, respectively. The line fitting step may use least-squares fitting, which has a closed-form solution and is fast, while assessing the goodness of the fit may use a robust loss function that rises linearly or slower than linearly with the error.

The number of inlier votes, or alternatively the value of the voting function, at the end of the RANSAC process can be used to derive a signal quality metric. Good signals will have a large ratio of inliers to the number of samples, while the ratio will decrease as the signal quality decreases.

According to an embodiment, an additional wavelength may be used only, which has a wavelength where both hemoglobin and oxyhemoglobin have high absorption. This gives a large signal amplitude and allows better recovery of the feature vector, i.e. this additional wavelength channel may not help with discriminating between the two hemoglobin modifications, but it gives a clear pulse signal.

According to another embodiment, non-uniform sampling of some or all of the light intensity channels may be used. This includes random non-uniform sampling, non-uniform sampling based on the plethysmographic waveform, and non-uniform sampling based on other means of estimating the cardiac cycle and pulse wave, for example by using information from other vital signs measurements (especially ECG) of the patient.

According to still another embodiment, a long (several seconds) set of wavelength data may be partitioned into short segments (between 0.1 and 1 second in length) and a segment estimation may be performed for each segment. This reduces the amount of data the final ratio estimation algorithm has to process and speeds up the computation.

According to still another embodiment, employ several different methods for determining the ratio may be employed. This may include conventional methods (e.g. peak-valley search, variance/standard deviation, frequency domain-based methods) in addition to at least one of the options described herein. This embodiment may use the degree of discrepancy between the output SpOz values of the different options as a measurement quality indicator, as for good signals the differences will be small, while for poor quality signals large differences may occur.

According to still another embodiment, a quality metric may be derived in addition to the ratio/$SpO_2$ value. In case of line fitting/line detection (including Hough transform and RANSAC), the quality metric may use the residual error values (differences between the fitted line and the data points). For PCA, the ratio of the eigenvalues of the covariance matrix to the largest eigenvalue can be used, as this ratio quantifies the power of the signal components other than the pulse signal. For PCA-like approaches (Lp-norm PCA, major direction search, ICA), the ratio of the variance of the non-pulse signal components to the variance of the pulse signal may be used to derive a quality metric.

The presented device, system and method allow the determination of more accurate physiological information, e.g. a more accurate ratio value, even in the presence of artefacts, noise and other undesired, nuisance signal components. The computation is less sensitive to artefacts and noise in the acquired light intensity signals. For instance, the ratio or $SpO_2$ value is more accurate when undesired signal components are present. More accurate SpOz values allow a better distinction between pathological and normal patient condition, and timely diagnosis and treatment of the former.

Additionally, since pulse oximeters usually perform additional processing of the individual $SpO_2$ values and may only present a value to the user if the sequence of SpOz value fulfils certain criteria for stability and plausibility, deriving a more accurate raw SpOz value will increase the availability of the SpOz measurement from the user's point of view. The increases the users' confidence in the pulse oximeter.

A comparison of several methods for finding the ratio, applied to a dataset with a large artefact, has revealed that the Hough transform/maximum likelihood approach and particularly the RANSAC approach result in a ratio very close to the theoretical ideal value, while the PCA approach is the least robust and shows a significant deviation from the ideal value. However, the result of a naive peak-valley approach deviates even more from the ideal value than the PCA approach.

Generally, according to the present invention, the problem of ratio calculation can be cast as a problem of line fitting, line/ridge detection or 3D plane fitting (to the data points in the three-dimensional (time, red, IR) space). Any algorithms for these classes of problems may be applied according to the present invention to address and solve this problem. Further, ratio detection may be recognized as a projection pursuit problem (similar to PCA, Lp-norm PCA, ICA), so that other algorithms for addressing and solving this problem may be used according to the present invention.

The invention can be used in pulse oximeters. Pulse oximeters can be a component of multi-parameter patient monitors but can also be stand-alone devices or integrated into other classes of devices, such as smart watches or other wearables. More generally, the invention can be used in any measurement of the ratio of constituents of the arterial blood that have different light absorption properties.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device (32, 52) for determining physiological information of a subject related to the subject's blood, the device comprising circuitry (33) configured to:

   - obtain two or more photoplethysmography, PPG, signals of the subject in two or more different wavelength channels;
   - determine the gradient or angle of a line in a two- or multi-dimensional coordinate system, in particular a cartesian coordinate system, fitting most PPG signal points in the coordinate system, wherein a PPG signal point is represented in the coordinate system by a tuple of PPG signal values of the two or more PPG signals acquired at substantially the same point in time; and
   - determine the physiological information from the determined gradient or angle.

2. Device according to claim 1, wherein the circuitry (33) is configured to use a statistical method to determine the gradient or angle of the line.

3. Device according to claim 1 or 2, wherein the circuitry (33) is configured to use one of the following methods to determine the one or more parameters of the line and/or the gradient or angle of the line:

   - line fitting;

   - principal component analysis, PCA;
   - Lp-norm PCA;
   - general projection pursuit using utility and/or penalty functions;
   - independent component analysis, ICA;
   - maximum likelihood estimation;
   - Hough transform; and
   - random sample consensus, RANSAC.

4. Device according to any preceding claim, wherein the circuitry (33) is configured to take the logarithm of the PPG values and to represent a PPG signal point in the coordinate system by a tuple of the logarithm of the PPG signal values of the two or more PPG signals acquired at substantially the same point in time.

5. Device according to any preceding claim, wherein the circuitry (33) is configured to

   - use the determined gradient or angle as ratio of ratios or determine the ratio of ratios from the determined gradient or angle; and
   - determine oxygen saturation of the subject's blood based on the ratio of ratios, in particular using a calibration table between ratio of ratios values and values of oxygen saturation.

6. Device according to any preceding claim, wherein the circuitry (33) is configured to pre-process the PPG signals by high-pass filtering and/or low-pass filtering before representing the PPG signal points in the coordinate system and determining the gradient or angle of the line.

7. Device according to any preceding claim, wherein the circuitry (33) is configured to non-uniformly sample the PPG signals to obtain the PPG signal values represented in the coordinate system.

8. Device according to any preceding claim, wherein the circuitry (33) is configured to partition the PPG signals into PPG signal segments and to determine the gradient or angle of a line in the coordinate system and to determine the physiological information per tuple of PPG signal segments for the two or more wavelength channels.

9. Device according to any preceding claim, wherein the circuitry (33) is configured to

   - determine the gradient or angle of the line by use of two or more different methods;
   - determine the physiological information from each of the determined gradients or angles; and
   - determine quality information indicating the quality of the determined physiological information based on the discrepancy of the physiological information values determined from the determined gradients or angles.

10. Device according to any preceding claim, wherein the circuitry (33) is configured to determine quality information indicating the quality of the determined physiological information based on

    - residual error values, or
    - a ratio of eigenvalues of a covariance matrix to a largest eigenvalue, or
    - a ratio of the variance of non-pulse signal components to a variance of a PPG signal.

11. Device according to any preceding claim, wherein the circuitry (33) is configured to determine oxygen saturation and/or information on pathological modifications of hemoglobin of the subject's blood from the determined slope gradient or angle.

12. System (30, 40, 50) for determining physiological information of a subject related to the subject's blood, the system comprising:

    - a light detector (31, 42, 51) configured to detect light in two or more different wavelength channels reflected from skin of a subject or transmitted through tissue of the subject in response and to generate two or more PPG signals from the detected light in the two wavelength channels; and
    - a device (32, 52) according to any preceding claim for determining the physiological information of the subject from the PPG signals.

13. System according to claim 12, wherein

    - the light detector is a camera (51) configured to remotely detect light from skin of the subject, or
    - the light detector is a light sensor (42) configured to be arranged in contact with or close to skin of the subject and the system further comprises a light emitter (41) configured to emit light in the two or more wavelength channels.

14. Method for determining physiological information of a subject related to the subject's blood, the method comprising:

    - obtaining two or more photoplethysmography, PPG, signals of the subject in two or more different wavelength channels;
    - determining the gradient or angle of a line in a two- or multi-dimensional coordinate system, in particular a cartesian coordinate system, fitting most PPG signal points in the coordinate system, wherein a PPG signal point is represented in the coordinate system by a tuple of PPG signal values of the two or more PPG signals acquired at substantially the same point in time; and
    - determining the physiological information from the determined gradient or angle.

15. Computer program comprising program code means for causing a computer to carry out the steps of the method as claimed in claim 13 when said computer program is carried out on the computer.

FIG.1

FIG.2

FIG.3

FIG.4

FIG.5

FIG.6

FIG.7

FIG.8

FIG.9

FIG.10

FIG.11

Europäisches
Patentamt

European
Patent Office

Office européen
des brevets

# EUROPEAN SEARCH REPORT

**Application Number**

**EP 23 19 4560**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 6 987 994 B1 (MORTZ MARGARET S [US])<br>17 January 2006 (2006-01-17)<br>* abstract *<br>* column 1, line 26 – column 3, line 42 *<br>* column 3, line 59 – column 11, line 38 *<br>* figures 1,2,6,7,9 * | 1-6,<br>10-15<br>7,9 | INV.<br>A61B5/024<br>A61B5/1455<br>A61B5/00 |
| X | US 2011/098933 A1 (OCHS JAMES [US])<br>28 April 2011 (2011-04-28)<br>* abstract *<br>* paragraph [0002] – paragraph [0007] *<br>* paragraph [0024] – paragraph [0030] *<br>* paragraph [0042] *<br>* paragraph [0069] – paragraph [0101] *<br>* figures 1,2B,6A-B * | 1-3,5,6,<br>10-15 | |
| X | US 2004/267140 A1 (ITO KAZUMASA [JP] ET<br>AL) 30 December 2004 (2004-12-30)<br>* abstract *<br>* paragraph [0001] *<br>* paragraph [0004] – paragraph [0005] *<br>* paragraph [0010] *<br>* paragraph [0014] *<br>* paragraph [0035] – paragraph [0040] *<br>* paragraph [0088] – paragraph [0140] *<br>* figures 1,2B,3B,4,7-11 * | 1-6,8,<br>11-15 | TECHNICAL FIELDS SEARCHED (IPC)<br><br>A61B |
| X | WO 2013/166168 A1 (NELLCOR PURITAN BENNETT<br>IE [IE]; ADDISON PAUL [GB] ET AL.)<br>7 November 2013 (2013-11-07)<br>* abstract *<br>* page 4, line 10 – page 7, line 6 *<br>* page 9, line 11 – page 13, line 33 *<br>* figures 2,6 * | 1-5,<br>11-15 | |

-/--

The present search report has been drawn up for all claims

| Place of search | Date of completion of the search | Examiner |
|---|---|---|
| The Hague | 6 February 2024 | Van der Haegen, D |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**page 1 of 2**

Europäisches
Patentamt
European
Patent Office
Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/219882 A1 (HUIKU MATTI VELI TAPANI [FI] ET AL) 22 July 2021 (2021-07-22) * paragraph [0062] – paragraph [0066] * * paragraph [0087] * * figures 1,12 * | 1-3,5, 11-15 | |
| Y | WOLLING FLORIAN ET AL: "Unity in Diversity: Sampling Strategies in Wearable Photoplethysmography", IEEE PERVASIVE COMPUTING, IEEE SERVICE CENTER, LOS ALAMITOS, CA, US, vol. 18, no. 3, 1 July 2019 (2019-07-01), pages 63-69, XP011758645, ISSN: 1536-1268, DOI: 10.1109/MPRV.2019.2926613 [retrieved on 2019-11-26] * page 65 – page 66 * * figure 2 * | 7 | |
| Y | US 2010/016690 A1 (WATSON JAMES [GB] ET AL) 21 January 2010 (2010-01-21) * abstract * * paragraph [0004] – paragraph [0005] * * paragraph [0072] – paragraph [0087] * * figures 5,6 * | 9 | TECHNICAL FIELDS SEARCHED (IPC) |
| A | WO 2015/003938 A1 (KONINKL PHILIPS NV [NL]) 15 January 2015 (2015-01-15) * abstract * * figures 1,2 * | 13 | |

The present search report has been drawn up for all claims

| Place of search | Date of completion of the search | Examiner |
|---|---|---|
| The Hague | 6 February 2024 | Van der Haegen, D |

EPO FORM 1503 03.82 (P04C01)

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

## ANNEX TO THE EUROPEAN SEARCH REPORT
## ON EUROPEAN PATENT APPLICATION NO.

EP 23 19 4560

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2024

| Patent document cited in search report | | | Publication date | Patent family member(s) | | Publication date |
|---|---|---|---|---|---|---|
| US 6987994 | B1 | | 17-01-2006 | NONE | | |
| US 2011098933 | A1 | | 28-04-2011 | NONE | | |
| US 2004267140 | A1 | | 30-12-2004 | JP | 4632143 B2 | 16-02-2011 |
| | | | | JP | 2008188442 A | 21-08-2008 |
| | | | | US | 2004267140 A1 | 30-12-2004 |
| WO 2013166168 | A1 | | 07-11-2013 | US | 2013296659 A1 | 07-11-2013 |
| | | | | WO | 2013166168 A1 | 07-11-2013 |
| US 2021219882 | A1 | | 22-07-2021 | NONE | | |
| US 2010016690 | A1 | | 21-01-2010 | US | 2010016690 A1 | 21-01-2010 |
| | | | | US | 2014257061 A1 | 11-09-2014 |
| WO 2015003938 | A1 | | 15-01-2015 | CA | 2917635 A1 | 15-01-2015 |
| | | | | CN | 105377126 A | 02-03-2016 |
| | | | | EP | 3019078 A1 | 18-05-2016 |
| | | | | JP | 6449271 B2 | 09-01-2019 |
| | | | | JP | 2016528960 A | 23-09-2016 |
| | | | | MX | 361026 B | 26-11-2018 |
| | | | | RU | 2016104071 A | 15-08-2017 |
| | | | | US | 2017319114 A1 | 09-11-2017 |
| | | | | WO | 2015003938 A1 | 15-01-2015 |

EPO FORM P0459

For more details about this annex : see Official Journal of the European Patent Office, No. 12/82